# EUROPÄISCHE PATENTSCHRIFT

(11) **EP 3 758 769 B1**
(45) Veröffentlichungstag und Bekanntmachung des Hinweises auf die Patenterteilung: **18.06.2025**
(21) Anmeldenummer: 19711505.8
(22) Anmeldetag: 26.02.2019
(51) Int. Cl.: A61M 1/16, A61M 1/36

(54) **HALTEEINRICHTUNG ZUM HALTEN EINES OXYGENATORS UND EINER BLUTPUMPE**
HOLDING DEVICE FOR HOLDING AN OXYGENATOR AND A BLOOD PUMP
DISPOSITIF DE RETENUE DESTINÉ À RETENIR UN OXYGÉNATEUR ET UNE POMPE D'ASSISTANCE CIRCULATOIRE

(30) Priorität: 26.02.2018 DE 102018001466; 03.07.2018 LU 100850
(43) Veröffentlichungstag der Anmeldung: 06.01.2021
(73) Patentinhaber: Hemovent GmbH, 52076 Aachen (DE)
(72) Erfinder: LENZ, Christof, 90592 Schwarzenbruck (DE); MARSEILLE, Oliver, 52066 Aachen (DE); NOBIS, Andreas, 52146 Würselen (DE); NÖTZEL, Stefan, 52064 Aachen (DE); JOOST, Thilo, 61169 Friedberg (DE); HARTMANN, Benedict, 40237 Düsseldorf (DE); PAUL, Christof, 48165 Münster (DE)
(74) Vertreter: Sattler de Sousa e Brito, Clara
(86) Internationale Anmeldenummer: PCT/EP2019/054732
(87) Internationale Veröffentlichungsnummer: WO 2019/162526

(56) Entgegenhaltungen:
- US-A- 4 610 656
- US-A- 4 610 656
- US-A- 5 879 316
- US-A- 5 879 316
- US-A1- 2005 027 231
- US-A1- 2005 027 231
- US-B1- 6 306 346
- US-B1- 6 306 346
- US-B1- 6 306 346
- US-B2- 7 198 751
- US-B2- 7 198 751
- US-B2- 7 198 751
- US-B2- 7 846 122
- US-B2- 7 846 122
- US-B2- 7 846 122

## Beschreibung

Die Erfindung betrifft einen Koffer mit einer Halteeinrichtung zum Halten eines Oxygenators und einer Blutpumpe.

Außerdem betrifft die Erfindung eine Herz-Lungen-Maschine mit einem solchen Koffer. Patienten mit einem akuten Herzversagen oder einem akuten Lungenversagen können durch eine schnell eingesetzte Herz-Lungen-Unterstützung am Leben gehalten werden, um Zeit zu gewinnen, bis eine weitere Therapie eingeleitet werden kann. Hierbei wird der Patient durch geeignete Kanülen mit einem extrakorporalen Kreislauf verbunden, der die Herz- und Lungenfunktion gewährleistet.

Eine Herz-Lungen-Maschine besteht aus einer Blutpumpe und einer künstlichen Lunge. Das Blut wird durch einen peripheren oder zentralen Zugang mit Kanülen aus dem venösen Gefäßsystem entnommen und nach Anreicherung mit Sauerstoff auf der venösen oder arteriellen Seite wieder zugefügt. So werden die lebenswichtigen Organe mit frischem Blut versorgt, während der Patient transportiert oder einer weiteren Therapie zugeführt werden kann.

Die Blutpumpe ist in der Regel mit einer Antriebskonsole fest verbunden und durch eine Magnetkupplung wird die Antriebsenergie von einem Pumpenmotor auf einen Rotor der Blutpumpe übertragen. Auch werden Rollerpumpen eingesetzt, bei denen ein Pumpenschlauch in die Antriebskonsole eingelegt wird. Die künstliche Lunge, auch Oxygenator oder Gastauscher genannt, befindet sich in Flussrichtung hinter der Blutpumpe und wird in der Regel an einem Halter nahe der Blutpumpe fest fixiert. Dabei müssen der Oxygenator und die Blutpumpe in einem bestimmten Winkel zueinander angeordnet werden, um einen einwandfreien Betrieb der Herz-Lungen-Maschine sicherzustellen. Bei Notfallsituationen werden der Oxygenator und die Blutpumpe zum Patienten transportiert und dort vor Ort zueinander ausgerichtet und miteinander fluidisch verbunden, was sehr zeitaufwändig ist.

Zu und von der Antriebskonsole führen blutführende Schläuche zum Patienten. Diese Schläuche müssen eine hinreichende Länge aufweisen, damit der Patient sicher bewegt werden kann, ohne dass die Schläuche geknickt oder anders mechanisch belastet werden. Die körperfremde Oberfläche der Schläuche stellt dabei eine Belastung für das Blut dar, so dass möglichst kurze Schläuche erstrebenswert sind. Auch kommt es bei Transportsituationen durch die langen Schläuche und die Vielzahl der zu transportierenden Bestandteile der Herz-Lungen-Maschine zu lebensbedrohlichen Komplikationen, da der Patient und die oben genannten Bestandteile der Herz-Lungen-Maschine in der Regel getrennt voneinander bewegt werden.

Die US 7,846,122 B2 zeigt eine Herz-Lungen-Maschine, welche aus verschiedenen Modulen, nämlich einem Basismodul, einem Steuermodul und einem Patientenmodul, zusammengesetzt ist.

Aus den US 7,198,751 B2 und die US 6,306,346 B1 gehen weitere Herz-Lungen-Maschinen hervor, bei denen mindestens teilweise die einzelnen Komponenten der Herz-Lungen-Maschine um den Patienten angeordnet werden müssen. Die US 4 610 656 A beschreibt einen Koffer mit einer Halteeinrichtung zum Halten eines Oxygenators und einer Blutpumpe.

Somit besteht ein Nachteil bei den bekannten Herz-Lungen-Maschinen darin, dass bei Notfallsituationen unnötige Zeit vergeht, um den Oxygenator und die Blutpumpe zueinander auszurichten und der Transport der Bestandteile der Herz-Lungen-Maschine aufwändig ist.

Die Aufgabe der Erfindung besteht daher darin, eine schnellere Herz-Lungen-Unterstützung des Patienten mit akutem Lungenversagen- oder Herzstillstand zu ermöglichen und den Transport der Bestandteile der Herz-Lungen-Maschine zu vereinfachen.

Die Erfindung wird durch die Merkmale des unabhängigen Anspruchs 1 definiert.

Die Aufgabe wird durch einen Koffer mit einer Halteeinrichtung der eingangs genannten Art gelöst, die gekennzeichnet ist durch einen mit einem Koffergehäuse drehfest verbindbaren Rahmen zum Festlegen des Oxygenators und der Blutpumpe relativ zueinander, wobei der Rahmen einen Rahmenabschnitt zum drehfesten Verbinden mit dem Oxygenator und einen anderen Rahmenabschnitt zum drehfesten Verbinden mit der Blutpumpe aufweist.

Die erfindungsgemäße Halteeinrichtung weist den Vorteil auf, dass der nicht zur Halteeinrichtung gehörende Oxygenator und die nicht zur Halteeinrichtung gehörende Blutpumpe durch den Rahmen festgelegt sind, so dass eine Relativbewegung zwischen dem Oxygenator und der Blutpumpe verhindert wird. Dies bietet den Vorteil, dass der Oxygenator und die Blutpumpe vor einem Einsatz bei einem Patienten mit dem Rahmen befestigt werden können. Daher entfällt ein Ausrichten des Oxygenators und der Blutpumpe relativ zueinander bei dem Patienten, sodass eine Unterstützung des Patienten mit der Herz-Lungen-Maschine schnell erfolgen kann. Außerdem müssen der Oxygenator und die Blutpumpe nicht getrennt transportiert werden, sondern können durch die Halteeinrichtung gemeinsam transportiert werden, was den Transport vereinfacht. Ebenso ist ein Transport von Blutpumpe und Oxygenator direkt zusammen mit dem Patienten möglich.

Die Relativbewegung zwischen dem Oxygenator und der Blutpumpe wird dadurch verhindert, dass der Rahmen derart ausgebildet ist, dass der Rahmenabschnitt und der andere Rahmenabschnitt sich nicht relativ zueinander bewegen können. So können der Rahmenabschnitt und der andere Rahmenabschnitt stets miteinander verbunden sein. Dies kann beispielsweise dadurch realisiert werden, dass der Rahmenabschnitt, der andere Rahmenabschnitt und der restliche Rahmen einstückig ausgeführt sind.

Im Sinne der Erfindung wird als Oxygenator ein Gerät verstanden, mittels dem Blut mit Sauerstoff angereichert und Kohlenstoffdioxid aus dem Blut entfernt werden kann. Somit kann mittels des Oxygenators zumindest kurzfristig die Lunge ersetzt werden.

Die Verbindung des Rahmenabschnitts mit dem Oxygenator kann formschlüssig und/oder reibschlüssig erfolgen. Darüber hinaus kann die Verbindung des anderen Rahmenabschnitts mit der Blutpumpe formschlüssig und/oder reibschlüssig erfolgen. Auch kann der Oxygenator mit dem Rahmen stoffschlüssig verbunden, insbesondere verkleibt sein.

Erfindungsgemäß weist die Halteeinrichtung einen Handgriff auf, der mit dem Rahmen drehfest verbunden ist. Insbesondere können der Handgriff und der Rahmen einstückig ausgeführt sein und/oder stets miteinander verbunden sein. Dadurch wird der Transport des Oxygenators und der Blutpumpe weiter vereinfacht.

Der Handgriff kann eine Führung zum Führen von einer Fluidleitung oder von mehreren Fluidleitungen aufweisen. Dabei kann durch die Fluidleitung ein Fluid, wie ein Gas, insbesondere Sauerstoff oder ein Gasgemisch, oder Blut, strömen. Bei Vorsehen von mehreren Fluidleitungen kann durch wenigstens eine Leitung Blut und durch wenigstens eine andere Leitung Gas, insbesondere Sauerstoff oder ein Gasgemisch, strömen. Der Handgriff kann an einem Ende des Rahmens angeordnet sein. Insbesondere kann der Handgriff an dem vom Oxygenator und der Blutpumpe entfernten Ende des Rahmens angeordnet sein.

Die Führung kann derart ausgebildet sein, dass mehrere Fluidleitungen, insbesondere wahlweise, in die gleiche Richtung oder in unterschiedliche Richtungen geführt werden. Insbesondere können die Fluidleitungen durch die Führung in sich entgegengesetzte Richtungen geführt werden. Darüber hinaus kann die Führung eine Aussparung aufweisen, in der die Fluidleitung anordenbar ist oder in der die Fluidleitungen anordenbar sind.

Der Rahmen kann eine andere Führung zum Führen der Fluidleitung oder von mehreren Fluidleitungen aufweisen. Dabei kann der Rahmen die Fluidleitungen führen, durch die jeweils Blut strömt. Die andere Führung kann eine andere Aussparung aufweisen, in der die Fluidleitung anordenbar ist oder in der die Fluidleitungen anordenbar sind.

Durch das Vorsehen der Führung und/oder der anderen Führung kann auf einfache Weise verhindert werden, dass die Fluidleitung beschädigt wird oder die Fluidleitungen beschädigt werden. Außerdem können die Fluidleitungen durch zusätzliche lösbare Fixierungen im und am Rahmen befestigt werden. Die Fixierung kann formschlüssig erfolgen. Insbesondere können Gurte oder Clipse zum Fixieren der Fluidleitungen eingesetzt werden.

Bei einer besonderen Ausführung können der Oxygenator und die Blutpumpe durch den Rahmenabschnitt und den anderen Rahmenabschnitt derart angeordnet sein, dass der Abstand zwischen einem Oxygenatoreinlass und dem Handgriff kürzer ist als der Abstand zwischen einem Blutpumpeneinlass und dem Handgriff. Insbesondere kann der andere Rahmenabschnitt derart positioniert sein, dass eine Strömungsrichtung des aus der Blutpumpe ausströmenden Bluts zum Handgriff weist und/oder dass das Blut in eine zum Handgriff zugewandte Richtung strömt. Somit strömt im Betrieb der Blutpumpe und des Oxygenators das aus der Blutpumpe ausströmende Blut schräg nach oben.

Von einem besonderen Vorteil ist der erfindungsgemäße Koffer mit einer Halteeinrichtung, wobei die Halteeinrichtung mit einem Koffergehäuse drehfest verbunden ist und teilweise in dem Koffergehäuse angeordnet ist. Das Koffergehäuse bietet den Vorteil, dass die Halteinrichtung, insbesondere der mit der Halteeinrichtung drehfest verbundene Oxygenator und die Blutpumpe gut gegen Schock- und/oder Stoßbelastungen geschützt sind. Außerdem wirkt das Koffergehäuse als thermischer Isolator, so dass ein geringer Wärmeverlust auftritt. Darüber hinaus wirkt das Koffergehäuse als akustischer Isolator. Ein weiterer Vorteil ist, dass der Koffer nah am Patienten platziert werden kann und somit lange Schläuche vermieden werden können. Der Oxygenator und die Blutpumpe sind mit dem Rahmen drehfest verbunden.

Bei einer besonderen Ausführung kann das Koffergehäuse einen ersten Gehäuseabschnitt und/oder einen zweiten Gehäuseabschnitt aufweisen. Der erste Gehäuseabschnitt und der zweite Gehäuseabschnitt können wieder lösbar miteinander drehfest verbunden sein. Die wieder lösbare Verbindung zwischen dem ersten Gehäuseabschnitt und dem zweiten Gehäuseabschnitt kann eine form- und/oder reibschlüssige Verbindung sein.

Der erste Gehäuseabschnitt und/oder der zweite Gehäuseabschnitt können wenigstens teilweise, insbesondere vollständig, aus einem nicht transparenten Material ausgebildet sein. Dabei kann der erste Gehäuseabschnitt aus wenigstens einem Schaumstoff bestehen. Der zweite Gehäuseabschnitt kann aus wenigstens einem Schaumstoff, insbesondere dem gleichen Schaumstoff wie der erste Gehäuseabschnitt, bestehen. Der Schaumstoff des ersten und/oder zweiten Gehäuseabschnitts kann ein Hartschaum sein. Der Schaumstoff weist den Vorteil auf, dass der Oxygenator und die Blutpumpe gut gegen Schock- und/oder Stoßbelastungen geschützt sind. Außerdem ist Schaumstoff ein guter thermischer Isolator.

Der erste Gehäuseabschnitt kann eine erste Aufnahme und der zweite Gehäuseabschnitt kann eine zweite Aufnahme aufweisen. Die erste Aufnahme und die zweite Aufnahme können einen Hohlraum bilden, in dem ein Teil des Rahmens und/oder der Oxygenator und/oder die Blutpumpe angeordnet sind. Insbesondere kann ein Teil des Rahmens und/oder der Oxygenator und/oder die Blutpumpe in dem ersten Gehäuseabschnitt und dem zweiten Gehäuseabschnitt wenigstens teilweise eingebettet sein. Dadurch wird auf einfache Weise vermieden, dass sich der Rahmen und/oder der Oxygenator und/oder die Blutpumpe relativ zueinander und/oder der dem Koffergehäuse bewegen können.

Bei einer besonderen Ausführung kann zum teilweisen Freigeben des Oxygenators ein den Oxygenator aufnehmender Teil der zweiten Aufnahme als Durchbruch ausgeführt sein. Darüber hinaus kann zum teilweisen Freigeben der Blutpumpe ein die Blutpumpe aufnehmender anderer Teil der zweiten Aufnahme als Durchbruch ausgeführt sein. Außerdem kann ein den Rahmen aufnehmender weiterer Teil der zweiten Aufnahme als Durchbruch ausgeführt sein. Die Durchbrüche ermöglichen, dass ein Anwender überprüfen kann, ob der Oxygenator und/oder die Blutpumpe wirksam arbeitet, ohne dass der zweite Gehäuseabschnitt von dem ersten Gehäuseabschnitt gelöst werden muss.

Darüber hinaus kann der zweite Gehäuseabschnitt eine Ausnehmung aufweisen, die als Durchbruch ausgeführt sein kann. Die Ausnehmung kann derart ausgebildet sein, dass in ihr Ersatzteile und/oder eine Bedienungsanleitung und/oder Sensoren angeordnet sind.

Der Koffer kann einen Verschlussdeckel zum Verschließen des Teils der zweiten Aufnahme und/oder des anderen Teils der zweiten Aufnahme und/oder des weiteren Teils der zweiten Aufnahme und/oder der Ausnehmung aufweisen. Der Verschlussdeckel kann wieder lösbar mit dem zweiten Gehäuseabschnitt verbunden sein. Der Verschlussdeckel kann an der von dem ersten Gehäuseabschnitt abgewandten Seite des zweiten Gehäuseabschnitts angeordnet sein.

Durch den Verschlussdeckel wird auf einfache Weise verhindert, dass der durch den Teil der zweiten Aufnahme freigegebene Teil des Oxygenators und/oder der durch den anderen Teil der zweiten Aufnahme freigegebene Teil der Blutpumpe und/oder die im Rahmen verlaufenden Fluidleitungen beispielsweise beim Transport des Koffers beschädigt werden können. Darüber hinaus können bei einem abgenommenen Verschlussdeckel die Fluidleitungen auf einfache Weise mit der Blutpumpe und/oder dem Oxygenator fluidisch verbunden werden. Somit ist es nicht notwendig, die beiden Gehäuseabschnitte voneinander zu trennen, um die Fluidleitungen mit dem Oxygenator und/oder der Blutpumpe zu verbinden.

Bei einer besonderen Ausführung kann der erste Gehäuseabschnitt und/oder der zweite Gehäuseabschnitt eine Durchgangsöffnung aufweisen, durch die die wenigstens eine mit dem Oxygenator und/oder der Blutpumpe fluidisch verbundene Fluidleitung aus dem Koffergehäuse austritt. Alternativ können durch die Durchgangsöffnung mehrere mit dem Oxygenator und/oder der Blutpumpe fluidisch verbundene Fluidleitungen aus dem Koffer austreten. Darüber hinaus kann sich ein Teil des Rahmens durch die Durchgangsöffnung hindurch erstrecken. Die Durchgangsöffnung kann durch einen Abschnitt der ersten Aufnahme und/oder einen Abschnitt der zweiten Aufnahme gebildet sein.

Dabei können mehrere, insbesondere genau zwei, Fluidleitungen mit dem Patienten fluidisch verbunden sein. Darüber hinaus können andere Fluidleitungen mit einer Gasquelle, wie beispielsweise einer Sauerstoffflasche, fluidisch verbunden sein. Zwei Bauteile sind miteinander fluidisch verbunden, wenn ein Gas und/oder eine Flüssigkeit von einem Bauteil in das andere Bauteil oder umgekehrt strömen kann. Dem Koffer, insbesondere dem Oxygenator und/oder der Blutpumpe, kann ein Gas oder ein Gasgemisch, wie beispielsweise ein Gemisch aus Sauerstoff und Luft, zugeführt werden.

Der Koffer kann einen Fluidauslassdurchgang aufweisen, durch den ein Fluid, wie beispielsweise Kondenswasser, aus dem ersten Gehäuseabschnitt und/oder dem zweiten Gehäuseabschnitt ausströmbar ist. Dadurch kann auf einfache Weise verhindert werden, dass sich Wasser innerhalb des Koffers sammelt.

Der Koffer kann eine weitere Durchgangsöffnung aufweisen, durch die sich wenigstens eine Abzweigleitung zum Zuführen von Blut zu einer Analyseeinrichtung erstreckt. Durch die Abzweigleitung strömt Blut, das stromaufwärts oder stromabwärts von dem Oxygenator abgezweigt wurde und das einer Analyseeinrichtung zugeführt wird. In der Analyseeinrichtung können Blutgasanalysen durchgeführt werden.

Die Abzweigleitung und/oder die Fluidleitungen können in dem Koffergehäuse verstaut werden. Insbesondere können die erste Aufnahme und die zweite Aufnahme derart ausgebildet sein, dass sie die Abzweigleitung und die Fluidleitungen aufnehmen können. Zum Betrieb kann die Abzweigleitung und/oder die Fluidleitung aus dem Koffergehäuse teilweise herausgezogen werden. Die Abzweigleitung und/oder die Fluidleitungen können im Koffer mit wenigstens einem Rückholelement, insbesondere einem elastischen Element, verbunden sein. Das Rückholelement ist derart ausgebildet, dass es nach dem Herausziehen der Abzweigleitung und/oder der Fluidleitung eine Rückholkraft auf die Abzweigleitung und/oder die Fluidleitung ausübt, so dass diese nach der Benutzung wieder in das Koffergehäuse gezogen werden.

Dabei kann wenigstens eine Arretierung vorhanden sein, die mit der Fluidleitung und/oder der Abzweigleitung verbindbar sind. Die Arretierung kann am Kofferaußenrand angeordnet sein und dafür sorgen, dass die Leitungen bei Benutzung trotz Rückholkraft in Position bleiben und somit nicht in den Koffer gezogen werden. Die Abzweigleitung und/oder die Fluidleitungen können an einem Ende mit automatischen und abnehmbaren Entlüftungsventilen versehen sein und wiederverschließbar sein. Die Abzweigleitung kann wenigstens 20cm lang sein.

Der Koffer kann durch die Durchgangsöffnung und/oder die weitere Durchgangsöffnung und/oder durch den Fluidauslassdurchgang, insbesondere vor der Inbetriebnahme des Koffers, entlüftet werden. Dies kann automatisch erfolgen, wenn der Koffer beispielsweise auf einer horizontalen Ebene platziert ist.

Der Koffer kann eine Standfläche aufweisen, die derart angeordnet und ausgebildet ist, dass der Oxygenatoreinlass in vertikaler Richtung, insbesondere in Schwerkraftrichtung, oberhalb des Blutpumpenauslasses angeordnet ist, wenn der Koffer mittels der Standfläche auf eine Abstellfläche abgesetzt ist. Die Abstellfläche kann beispielsweise der Boden sein, auf den der Koffer beim Einsatz gesetzt wird. Im Ergebnis kann durch das Aufsetzen des Koffers über die Standfläche auf der Abstellfläche die Halteeinrichtung so ausgerichtet werden, dass der Oxygenatoreinlass oberhalb des Blutpumpenauslasses angeordnet ist.

Bei einer besonderen Ausführung kann die Blutpumpe stromlos angetrieben werden. Dies bedeutet, dass der Blutpumpe keine elektrische Energie zugeführt wird. Dabei kann die Blutpumpe mittels des in der Fluidleitung strömenden Gases angetrieben werden. Im Ergebnis wird ein Koffer realisiert, der nur mit einer Gasquelle und/oder einer Vorrichtung zum Steuern oder Regeln des dem Oxygenator und/oder Blutpumpe zugeführten Gasvolumenstroms verbunden werden muss, damit der Oxygenator und die Blutpumpe betrieben werden können.

Alternativ ist der Einsatz einer Blutpumpe möglich, die mittels Strom angetrieben wird. Dazu kann im Koffer wenigstens ein elektrischer Energiespeicher angeordnet sein, der die Blutpumpe mit elektrischer Energie versorgt. Alternativ kann die Blutpumpe mittels elektrischer Leitungen mit einer außerhalb des Koffers angeordneten Energiequelle elektrisch verbunden sein.

Die Blutpumpe kann als pulsatile Verdrängerpumpe oder als Rotationspumpe ausgeführt sein.

Die Blutpumpe und der Oxygenator können miteinander fluidisch verbunden sein. Dabei kann die Blutpumpe dem Oxygenator strömungstechnisch vorgeschaltet sein. Insbesondere kann das von dem Patienten abgenommenen Blut durch die Blutpumpe und anschließend durch den Oxygenator strömen. Von dem Oxygenator strömt das Blut zurück zum Patienten.

Ein Blutpumpeneinlass kann mit einer ersten Leitung der Fluidleitungen, insbesondere direkt, fluidisch verbunden sein. Ein anderes Ende der ersten Leitung kann mit dem Patienten, insbesondere direkt, fluidisch verbunden sein. Die Blutpumpe kann mittels einer zweiten Leitung der Fluidleitungen mit dem Oxygenator fluidisch verbunden sein. Ein Oxygenatorausgang kann mittels einer dritten Leitung der Fluidleitungen, insbesondere direkt, mit dem Patienten fluidisch verbunden sein.

Eine vierte Leitung der Fluidleitungen kann an einem Ende mit dem Oxygenator fluidisch verbunden sein. Darüber hinaus kann die vierte Leitung an einem anderen Ende mit einer Gasquelle fluidisch verbunden sein. Die Gasquelle kann eine stationäre Gasversorgung, eine Gasflasche oder ein pneumatisches Steuerwerk sein. Wie oben bereits beschrieben wurde, kann die Blutpumpe stromlos angetrieben werden. Dazu kann die Blutpumpe mit wenigstens einer fünften Leitung der Fluidleitungen, insbesondere mehrere fünfte Leitungen, fluidisch verbunden sein. Die fünfte Leitung kann an einem anderen Ende mit der Gasquelle fluidisch verbunden sein.

Die vierte Leitung und die fünfte Leitung können mit derselben Gasquelle fluidisch verbindbar sein. Darüber hinaus können die vierte Leitung und die fünfte Leitung mit derselben Vorrichtung zum Steuern oder Regeln eines Gasvolumenstroms in der vierten Leitung und/oder der fünften Leitung bzw. den fünften Leitungen verbindbar sein. Insbesondere kann die Vorrichtung mit der vierten Leitung und/oder der fünften Leitung fluidisch verbindbar sein.

Bei dem Koffer ist der Handgriff außerhalb des Koffergehäuses angeordnet Somit kann der Koffer mittels des Handgriffs auf einfache Weise getragen werden. Im Ergebnis erfüllt die Halteeinrichtung mehrere Funktionen, wie beispielsweise ein Tragen des Koffers zu ermöglichen, den Oxygenator und die Blutpumpe fest zueinander auszurichten, in den Koffer ein- und austretende Fluidleitungen zu schützen und eine Richtung zu geben und eine Relativbewegung zwischen dem Oxygenator und der Blutpumpe zu verhindern.

Der Koffer kann eine Befestigungseinrichtung zum Befestigen des Koffers mit einem Gegenstand, wie beispielsweise einem Krankenbett aufweisen. Die Befestigungseinrichtung kann auf einer Seite mit dem Koffer, insbesondere dem Koffergehäuse, und/oder dem darin einliegenden Rahmen der Halteeinrichtung und auf einer anderen Seite mit dem Gegenstand fest oder beweglich verbunden sein.

Die Befestigungsvorrichtung kann eine Halterung und eine Halterungsaufnahme aufweisen. Die Halterung kann in einer Stellung in der Halterungsaufnahme angeordnet sein. Bei dieser Stellung ist der Koffer nicht mit dem Krankenbett verbunden. Darüber hinaus kann die Halterung in einer anderen Stellung aus der Halterungsaufnahme teilweise hervorstehen. Bei dieser Stellung kann der Koffer mit dem Krankenbett verbunden werden. Die Halterung kann an ihren Enden jeweils hackenförmig ausgebildet sein. Die beiden hackenförmigen Enden können durch einen Zwischensteg miteinander verbunden sein, wobei der Zwischensteg einen u-förmigen Abschnitt aufweist. Der Zwischensteg wird beim Überführen der Halterung von der Stellung in die andere Stellung um 90° gedreht werden. Die Halterung kann ausgehend von der anderen Stellung nicht weiter gedreht werden. Ein weiteres Drehen der Halterung wird dadurch verhindert, dass der u-förmige Abschnitt gegen eine Platte der Befestigungsvorrichtung anstößt und somit ein weiteres Drehen der hackenförmigen Enden auf einfache Weise vermieden wird.

Die Halterung kann beweglich mit dem Koffergehäuse und/oder dem Rahmen verbunden sein und insbesondere durch klappen, schwenken oder stecken aus der Halterungsaufnahme herausgeführt werden. Im Ergebnis kann der Koffer auf einfache Weise mit dem Krankenbett verbunden werden.

Der Koffer kann steril ausgebildet sein. Bei dieser Ausführung können die Halteeinrichtung, der Oxygenator und die Blutpumpe durch den Benutzer des Koffers nicht ausgetauscht werden können.

Alternativ kann der Koffer ein Set von gegeneinander auswechselbaren Oxygenatoren und/oder ein Set von gegeneinander auswechselbaren Blutpumpen und/oder ein Set von gegeneinander auswechselbaren Halteeinrichtungen aufweisen. Bei diesem Fall kann der Benutzer den Koffer vorkonfigurieren. Insbesondere kann der Benutzer einen einzigen Oxygenator aus dem Set von Oxygenatoren, eine einzige Blutpumpe aus dem Set von Blutpumpen und eine einzige Halteeinrichtung aus dem Set von Halteeinrichtungen auswählen. Der ausgewählte Oxygenator und die ausgewählte Blutpumpe werden mit der ausgewählten Halteeinrichtung verbunden. Diese Ausführung bietet den Vorteil, dass der Koffer flexibler einsetzbar ist.

Der Koffer kann eine Bestimmungseinrichtung, insbesondere eine Wasserwaage, zum Bestimmen einer Ausrichtung des Koffers aufweisen. Die Bestimmungseinrichtung kann in einer Aussparung des Koffergehäuses angeordnet sein. Insbesondere kann die Bestimmungseinrichtung in einer im ersten Gehäuseabschnitt und/oder zweiten Gehäuseabschnitt vorhandenen Aussparung angeordnet sein. Mittels der Bestimmungseinrichtung kann die Ausrichtung des Koffers in wenigstens einer Richtung, insbesondere in zwei Richtungen bestimmt werden. Dabei kann mittels der Bestimmungseinrichtung die Ausrichtung des Koffers in horizontaler Richtung ermittelt werden. Durch Vorsehen der Bestimmungseinrichtung ist es auf einfache Weise möglich, sicherzustellen, dass der Koffer beim Einsatz auf einer horizontalen Ebene abgelegt wird.

Dies ist wichtig, um sicherzustellen, dass beim Einsatz der Oxygenatoreinlass in vertikaler Richtung oberhalb eines Blutpumpenauslasses angeordnet ist.

Von besonderem Vorteil ist eine Herz-Lungen-Maschine mit einem erfindungsgemäßen Koffer, der Gasquelle und der Vorrichtung zum Steuern oder Regeln, die einen Volumenstrom des zu dem Oxygenator und der Blutpumpe zugeführten Gases steuert oder regelt. Die Vorrichtung kann strömungstechnisch zwischen der Gasquelle und dem Koffer angeordnet sein und/oder mit der Gasquelle und dem Koffer fluidisch verbunden sein.

In den Figuren ist Erfindungsgegenstand schematisch dargestellt und wird anhand der Figuren nachfolgend beschrieben, wobei gleiche oder gleichwirkende Elemente zumeist mit denselben Bezugszeichen versehen sind. Dabei zeigen:
- Fig. 1: eine Darstellung einer erfindungsgemäßen Halteeinrichtung,
- Fig. 2: eine Darstellung der erfindungsgemäßen Halteeinrichtung von schräg oben,
- Fig. 3: eine Darstellung eines erfindungsgemäßen Koffers gemäß einem ersten Ausführungsbeispiel,
- Fig. 4: eine Darstellung eines ersten Gehäuseabschnitts des erfindungsgemäßen Koffers gemäß dem ersten Ausführungsbeispiel,
- Fig. 5: eine Darstellung eines zweiten Gehäuseabschnitts des erfindungsgemäßen Koffers gemäß dem ersten Ausführungsbeispiel,
- Fig. 6: eine Darstellung des erfindungsgemäßen Koffers gemäß dem ersten Ausführungsbeispiel ohne Verschlussdeckel,
- Fig. 7: eine Seitenschnittdarstellung eines erfindungsgemäßen Koffers gemäß einem zweiten Ausführungsbeispiel mit mehreren Fluidleitungen,
- Fig. 8: einen Einsatz des erfindungsgemäßen Koffers gemäß dem zweiten Ausführungsbeispiel in einem Krankenzimmer,
- Fig. 9: einen Einsatz des erfindungsgemäßen Koffers gemäß dem zweiten Ausführungsbeispiel bei einer Notfallsituation,
- Fig. 10: eine Darstellung eines Teils eines erfindungsgemäßen Koffers gemäß einem dritten Ausführungsbeispiel, wobei sich eine Halterung einer Befestigungsvorrichtung in einer eingeklappten Stellung befindet,
- Fig. 11: eine Darstellung des erfindungsgemäßen Koffers gemäß dem dritten Ausführungsbeispiel, wobei sich die Halterung in einer ausgeklappten Stellung befindet.

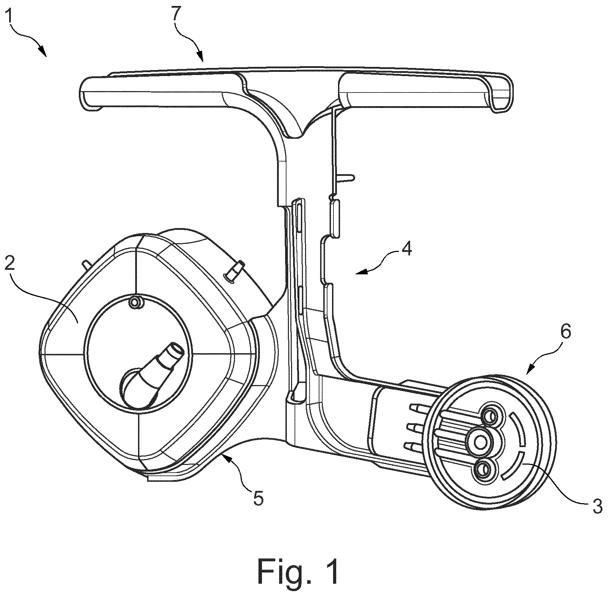

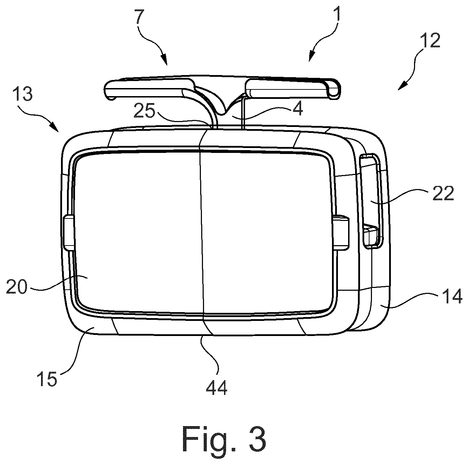

Eine in Figur 1 gezeigte Halteeinrichtung 1 zum Halten eines Oxygenators 2 und einer Blutpumpe 3 weist einen Rahmen 4 auf. Der Rahmen 4 kann mit einem in Figur 3 gezeigten Koffergehäuse 13 drehfest verbunden werden und dient zum Festlegen des Oxygenators 2 und der Blutpumpe 3 relativ zueinander. Der Rahmen 4 weist einen Rahmenabschnitt 5 und einen anderen Rahmenabschnitt 6 auf. Der Oxygenator 2 ist mit dem Rahmenabschnitt 5 drehfest verbunden und die Blutpumpe 3 ist mit dem anderen Rahmenabschnitt 6 drehfest verbunden.

Die Halteeinrichtung 1 weist außerdem einen Handgriff 7 auf, der mit dem Rahmen 4 drehfest verbunden ist. Insbesondere ist der Handgriff 7 mit dem Rahmen 4 einstückig ausgeführt. Dabei ist der Handgriff 7 mit einem Ende des Rahmens 4 drehfest verbunden. Der Rahmenabschnitt 5 und der andere Rahmenabschnitt 6 sind an einem anderen Ende des Rahmens 4 angeordnet.

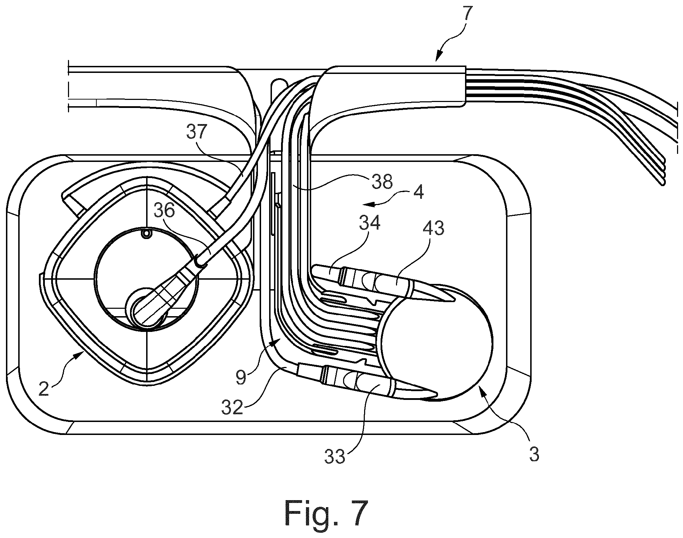

Der Rahmenabschnitt 5 und der andere Rahmenabschnitt 6 sind derart angeordnet, dass ein Oxygenatoreinlass näher zu dem Handgriff 7 angeordnet ist als ein in Figur 7 gezeigter Blutpumpenauslass 43, wobei das aus dem Blutpumpenauslass 43 ausgeströmte Blut über den Oxygenatoreinlass in den Oxygenator 2 einströmt. Somit ist der Blutpumpenauslass 43 von dem Handgriff 7 in vertikaler Richtung gesehen weiter weg angeordnet als der Oxygenatoreinlass 42, so dass die Richtung des aus der Blutpumpe 3 ausströmende Bluts schräg nach oben weist. Insbesondere ist die Blutpumpe 3 derart angeordnet, dass das aus der Blutpumpe 3 austretende Blut in eine zum Handgriff 7 zugewandte Richtung strömt. Die Blutstromrichtung steigt zwischen der Blutpumpe 3 und dem Oxygenator 2 in vertikaler Richtung an.

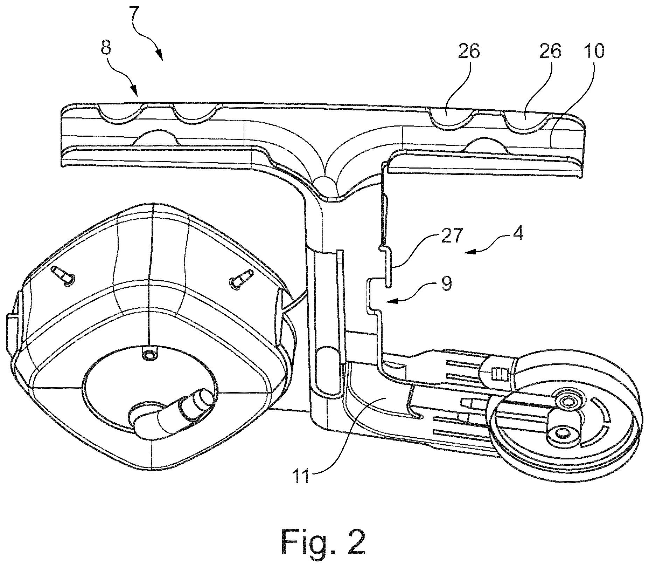

Figur 2 zeigt die Halteeinrichtung 1 aus einem anderen Blickwinkel als Figur 1. Wie aus Figur 2 ersichtlich ist, weist der Handgriff 7 eine Führung 8 auf. Die Führung 8 dient zum Führen von in Figur 7 dargestellten Fluidleitungen und weist eine Aussparung 10 auf, in der die in Figur 7 gezeigten Fluidleitungen angeordnet sind. Darüber hinaus weist die Führung 8 mehrere Vorsprünge 26 auf, die nach radial innen vorstehen und ein Herausfallen der Fluidleitungen aus der Führung 8 verhindern.

Der Rahmen 4 der Halteeinrichtung 1 weist eine andere Führung 9 zum Führen von wenigstens einer Fluidleitung auf. Die andere Führung 9 weist eine andere Aussparung 11 auf, in der die wenigstens eine Fluidleitung angeordnet ist. Die andere Führung 9 weist Seitenwände 27 auf, die ein Herausfallen der Fluidleitung aus der anderen Führung 9 verhindern.

Figur 3 zeigt einen erfindungsgemäßen Koffer 12 mit der Halteinrichtung 1, die mit einem Koffergehäuse 13 drehfest verbunden und teilweise in dem Koffergehäuse 13 angeordnet ist. Wie aus Figur 3 ersichtlich ist, ist der Handgriff 7 außerhalb des Koffergehäuses 13 angeordnet. Darüber hinaus ragt ein Teil des Rahmens 4 durch eine Durchgangsöffnung 25 aus dem Koffergehäuse 13 hervor.

Das Koffergehäuse 13 weist einen ersten Gehäuseabschnitt 14 und einen zweiten Gehäuseabschnitt 15 auf, die miteinander drehfest verbunden sind. Der Koffer 12 weist außerdem einen Verschlussdeckel 20 auf, der einen Teil des zweiten Gehäuseabschnitts 15 verdeckt.

An einer Seite des Koffers 12 ist eine weitere Durchgangsöffnung 22 vorgesehen. Durch die weitere Durchgangsöffnung 22 kann sich eine nicht dargestellte Abzweigleitung erstrecken, die an einem Ende mit einer nicht dargestellten Analyseeinrichtung fluidisch verbunden ist. In der Analyseeinrichtung kann das vom Patienten entnommene Blut analysiert werden. Das andere nicht dargestellte Ende der Abzweigleitung ist mit einem Anschluss verbunden, mittels dem Blut abgezweigt werden kann.

Der Koffer 12 weist eine Standfläche 44 auf, über die der Koffer 12 auf eine Abstellfläche, wie beispielswiese einen Boden, aufgesetzt wird. Die Standfläche 44 ist bezogen auf eine Horizontalebene an dem dem Handgriff 7 gegenüberliegenden Ende des Koffers 12 angeordnet. Beim Aufsetzen des Koffers 12 über die Standfläche 44 auf dem Boden ist der Koffer 12 so ausgerichtet, dass der Oxygenatoreinlass in vertikaler Richtung oberhalb des Blutpumpenauslasses angeordnet ist.

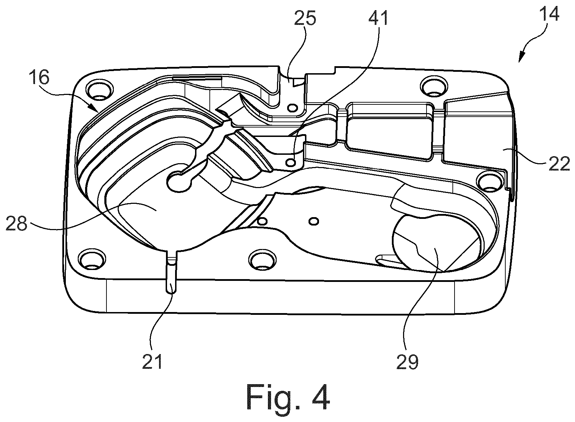

Figur 4 zeigt eine perspektivische Darstellung auf den ersten Gehäuseabschnitt 14. Der erste Gehäuseabschnitt 14 weist eine erste Aufnahme 16 auf. Die erste Aufnahme 16 dient zum Aufnehmen eines Teils des Rahmens 4, des Oxygenators 2 und der Blutpumpe 3. Dazu weist die erste Aufnahme 16 einen Teil 28 auf, der zum Aufnehmen des Oxygenators 2 dient. Außerdem weist die erste Aufnahme 16 einen anderen Teil 29 auf, der zur Aufnehmen der Blutpumpe 3 dient. Darüber hinaus weist die erste Aufnahme 16 einen weiteren Teil 41 auf, der zum Aufnehmen eines Teils des Rahmens 4 dient.

Der erste Gehäuseabschnitt 14 begrenzt zusammen mit dem zweiten Gehäuseabschnitt 15 die Durchgangsöffnung 25 und die weitere Durchgangsöffnung 22. Darüber hinaus begrenzt der erste Gehäuseabschnitt 14 zusammen mit dem zweiten Gehäuseabschnitt 15 einen Fluidauslassdurchgang 21. Der gemeinschaftlich mit dem zweiten Gehäuseabschnitt gebildete Fluidauslassdurchgang 21 ermöglicht, dass ein Fluid, wie beispielsweise Kondenswasser, aus dem Koffergehäuse 13 ausströmen kann.

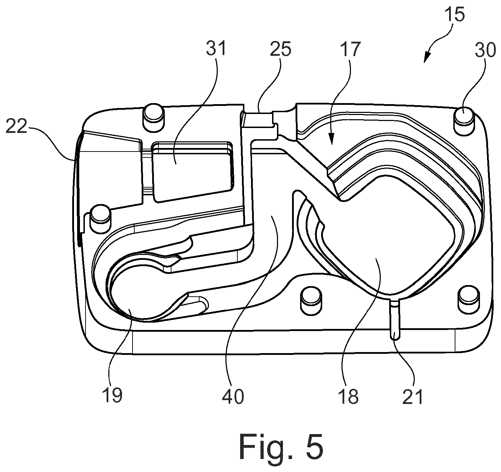

Figur 5 zeigt eine perspektivische Darstellung auf den zweiten Gehäuseabschnitt 15. Der zweite Gehäuseabschnitt 15 weist eine zweite Aufnahme 17 auf. Die zweite Aufnahme 17 dient zum Aufnehmen eines Teils des Rahmens 4, des Oxygenators 2 und der Blutpumpe 3. Dazu weist die zweite Aufnahme 17 einen Teil 18 auf, der zum Aufnehmen des Oxygenators 2, und einen anderen Teil 19 auf, der zum Aufnehmen der Blutpumpe 3 dient. Sowohl der Teil 18 als auch der andere Teil 19 der zweiten Aufnahme 17 sind als Durchbruch ausgebildet. Darüber hinaus ist der den Teil des Rahmens 4 aufnehmende weitere Teil 40 der zweiten Aufnahme 17 als Durchbruch ausgeführt.

Der zweite Gehäuseabschnitt 15 weist mehrere Hervorhebungen 30 auf, die zum Verbinden mit dem ersten Gehäuseabschnitt 14 in entsprechende im ersten Gehäuseabschnitt 14 vorgesehene Löcher eindringen. Darüber hinaus weist der zweite Gehäuseabschnitt 15 eine Ausnehmung 31 auf, die ebenfalls als Durchbruch ausgeführt ist.

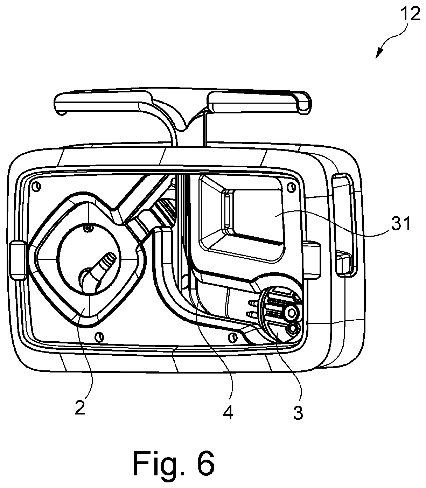

Figur 6 zeigt einen Koffer 12 ohne den Verschlussdeckel 20. Wie aus Figur 6 ersichtlich ist, sind aufgrund der zuvor beschriebenen Ausbildung der zweiten Aussparung 17 ein Teil des Oxygenators 2, ein Teil der Blutpumpe 3 und ein Teil des Rahmens 4 freigegeben. Dies bedeutet, dass die Bauteile von außen sichtbar sind. In der Ausnehmung 31 können Ersatzteile und/oder Sensoren und/oder die Abzweigleitung angeordnet sein.

Die erste Aufnahme 16 des ersten Gehäuseabschnitts 14 und die zweite Aufnahme 17 des zweiten Gehäuseabschnitts 15 bilden einen Hohlraum, in dem der Oxygenator 2, ein Teil des Rahmens 4 und die Blutpumpe 3 derart angeordnet sind, dass sie sich nicht relativ zueinander und/oder nicht relativ zu dem Koffergehäuse 13 bewegen können.

Figur 7 zeigt eine Seitenschnittdarstellung des Koffers 12 gemäß einer zweiten Ausführung mit mehreren Fluidleitungen. Der Schnitt erfolgt dabei derart, dass das Innere des Koffergehäuses 13 sichtbar ist. Wie aus Figur 7 ersichtlich ist, sind der Oxygenator 2 und die Blutpumpe 3 jeweils mit mehreren Fluidleitungen fluidisch verbunden.

So weisen die Fluidleitungen eine erste Leitung 32 auf, die an einem Ende mit einem Blutpumpeneinlass 33 fluidisch verbunden ist. Die erste Leitung 32 ist an einem anderen Ende mit dem Patienten fluidisch verbunden. Eine zweite Leitung 34 der Fluidleitungen ist an einem Ende mit einem Blutpumpenauslass 43 fluidisch verbunden. Zudem ist die zweite Leitung 34 an einem anderen Ende mit dem Oxygenator 2, insbesondere mit dem Oxygenatoreinlass, fluidisch verbunden. Das in den Oxygenator 2 eingeströmte Blut strömt nach dem Gasaustausch über eine dritte Leitung 36 zu dem Patienten zurück.

Darüber hinaus ist der Oxygenator 2 mit einer vierten Leitung 37 fluidisch verbunden. Dabei wird dem Oxygenator 2 mittels der vierten Leitung 37 Gas zugeführt. Dabei ist ein Ende der vierten Leitung 37 mit einer in Figur 3 nicht dargestellten Gasquelle, wie beispielsweise einem Gastank oder einer pneumatischen Steuerkonsole, fluidisch verbunden.

Die Blutpumpe 3 ist mit drei fünften Leitungen 38 fluidisch verbunden. Mittels der fünften Leitungen 38 wird der Blutpumpe 3 Gas, wie beispielsweise Sauerstoff oder Luft, zugeführt oder von dieser abgeführt. Durch das Zuführen oder Abführen des Gases kann eine Pumpwirkung bewirkt werden, die letztendlich den Blutvolumenstrom durch den Oxygenator 2 bewirkt.

Bei dem in Figur 7 dargestellten Koffer 12 werden alle Fluidleitungen durch den Handgriff 7 geführt. Dabei erstrecken sich alle Fluidleitungen in die gleiche Richtung. Die drei fünften Leitungen 38 werden durch die andere Führung 9 des Rahmens 4 geführt.

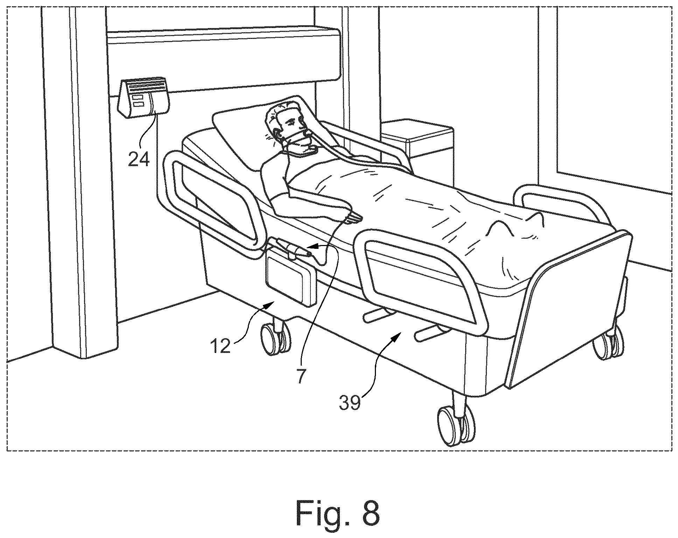

Figur 8 zeigt den Einsatz des in Figur 3 dargestellten Koffers 12 in einem Krankenzimmer. Der Koffer 12 ist an ein Krankenbett 39 angebracht. Im Krankenbett 39 liegt ein Patient, von dem Blut abgenommen wird, das zum Koffer 12 strömt. Das in dem Oxygenator 2 mit Sauerstoff angereicherte Blut strömt zum Patienten zurück.

Der Koffer 12 ist außerdem mittels der Fluidleitungen mit einer Vorrichtung 24 zum Steuern oder Regeln eines Gasvolumenstroms in den Fluidleitungen fluidisch verbunden. Durch Steuern oder Regeln des Gasvolumenstroms in den Fluidleitungen kann der durch die Blutpumpe 3 geförderte Blutvolumenstrom und der dem Oxygenator 2 zugeführte Gasvolumenstrom gesteuert oder geregelt werden. Die Vorrichtung 24 ist bei diesem Einsatzbereich an einer Wand des Krankenzimmers angebracht und über Fluidleitungen mit dem Oxygenator 2 und/oder der Blutpumpe 3 fluidisch verbunden. Die Vorrichtung 24 ist mit wenigstens einer nicht dargestellten Gasquelle fluidisch verbunden.

Die in Figur 8 dargestellte Ausführung unterscheidet sich von der in Figur 7 dargestellten Ausführung dadurch, dass sich die mit dem Patienten fluidisch verbundenen Fluidleitungen vom Handgriff 7 in eine andere Richtung erstrecken als die mit der Vorrichtung 24 verbundenen anderen Fluidleitungen. Auch die mit dem Patienten fluidisch verbundenen Fluidleitungen können sich in verschiedene Richtungen erstrecken, wenn der Patientenzugang an verschiedenen Körperstellen erfolgt.

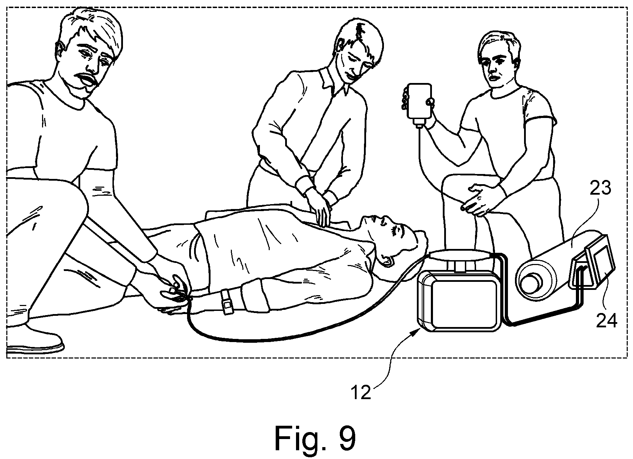

Figur 9 zeigt den Einsatz des in Figur 3 dargestellten Koffers 12 bei einer Notfallsituation, bei der bei einem am Boden liegenden Patienten eine akute Herz- oder Lungeninsuffizienz eingetreten ist. Der Koffer 12 ist auf dem Boden platziert. Aus Figur 9 ist analog zu Figur 8 ersichtlich, dass der Koffer 12 über jeweilige Fluidleitungen mit dem Patienten fluidisch verbunden sind. Darüber hinaus ist aus Figur 9 ersichtlich, dass der Koffer 12 mit der Vorrichtung zum Steuern oder Regeln 24 fluidisch verbunden ist. Die Vorrichtung zum Steuern oder Regeln 24 ist mit der Gasquelle 23 fluidisch verbunden, wobei die Vorrichtung 24 strömungstechnisch zwischen der Gasquelle 23 und dem Koffer 12 angeordnet ist.

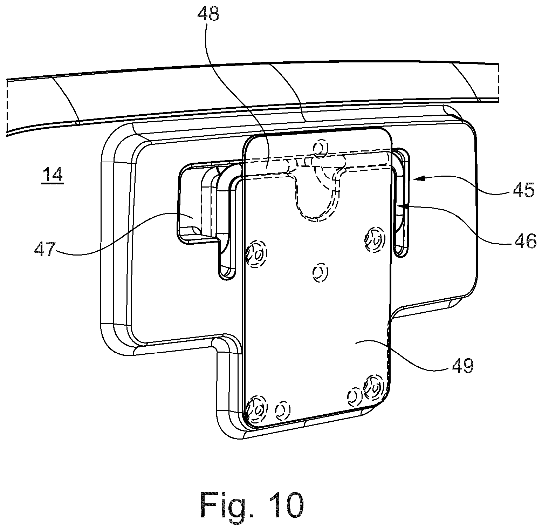

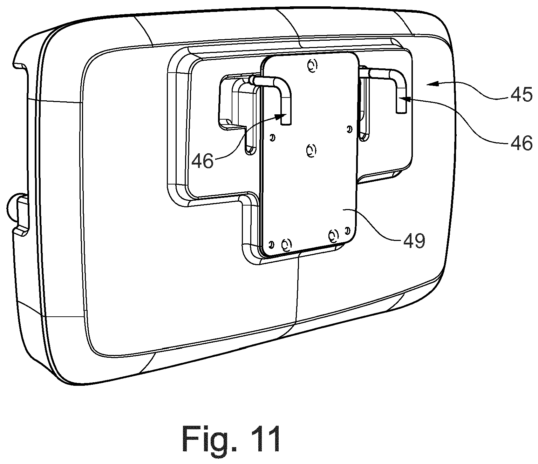

Figur 10 zeigt eine Darstellung eines Teils eines erfindungsgemäßen Koffers 12 gemäß einem dritten Ausführungsbeispiel. Der Koffer 12 weist eine Befestigungsvorrichtung 45 auf, die am ersten Gehäuseabschnitt 14 angebracht ist. Die Befestigungsvorrichtung 45 weist eine Halterung 46 und eine Halterungsaufnahme 47 auf. Die Halterung 46 ist, wie aus Figur 11 ersichtlich ist, an ihren beiden Enden hackenförmig ausgebildet. Die beiden hackenförmigen Enden sind durch einen Zwischensteg 48 miteinander verbunden. Der Zwischensteg 48 weist mittig einen u-förmigen Abschnitt auf. Der Zwischensteg 48 ist in Figur 10 ersichtlich, weil eine Platte 49 der Befestigungsvorrichtung 45 teilweise transparent dargestellt ist. Die Platte 49 deckt einen Teil der Halterungsaufnahme 47 ab.

Bei der in Figur 10 dargestellten Stellung der Halterung 46 ist diese in der Halterungsaufnahme 47 angeordnet. Bei dieser Stellung der Halterung 46 kann der Koffer 12 nicht mit dem Krankenbett verbunden werden.

Figur 11 zeigt den Koffer 12 gemäß der dritten Ausführung, wobei sich die Halterung 46 in einer ausgeklappten Stellung befindet. Die hackenförmigen Enden der Halterung 46 stehen aus der Halterungsaufnahme 47 und dem ersten Gehäuseabschnitt 14 jeweils hervor und können mit dem nicht dargestellten Krankenbett verbunden werden können.

Zum Überführen der Halterung 46 aus der in Figur 10 gezeigten eingeklappten Stellung in die in Figur 11 gezeigte ausgeklappte Stellung wird die Halterung 46 in einer Richtung gedreht. Ein weiteres Drehen der Halterung in dieser Richtung ist nicht möglich, weil der u-förmige Abschnitt des Zwischenstegs 48 gegen die Platte 49 stößt und somit ein weiteres Drehen der Halterung 46 verhindert. Der u-förmige Abschnitt ist bei der in Figur 11 dargestellten Stellung der Halterung 46 in der in Figur 10 ersichtlichen Ausnehmung angeordnet.

### Bezugszeichenliste:

- 1: Halteeinrichtung
- 2: Oxygenator
- 3: Blutpumpe
- 4: Rahmen
- 5: Rahmenabschnitt
- 6: anderer Rahmenabschnitt
- 7: Handgriff
- 8: Führung
- 9: andere Führung
- 10: Aussparung
- 11: andere Aussparung
- 12: Koffer
- 13: Koffergehäuse
- 14: erste Gehäuseabschnitt
- 15: zweite Gehäuseabschnitt
- 16: erste Aufnahme
- 17: zweite Aufnahme
- 18: Teil der zweiten Aufnahme
- 19: anderer Teil der zweiten Aufnahme
- 20: Verschlussdeckel
- 21: Fluidauslassdurchgang
- 22: weitere Durchgangsöffnung
- 23: Gasquelle
- 24: Vorrichtung zum Steuern oder Regeln
- 25: Durchgangsöffnung
- 26: Vorsprung
- 27: Seitenwand
- 28: Teil der ersten Aufnahme
- 29: anderer Teil der ersten Aufnahme
- 30: Hervorhebung
- 31: Ausnehmung
- 32: erste Leitung
- 33: Blutpumpeneinlass
- 34: zweite Leitung
- 36: dritte Leitung
- 37: vierte Leitung
- 38: fünfte Leitung
- 39: Krankenbett
- 40: weiterer Teil der zweiten Aufnahme
- 41: weiterer Teil der ersten Aufnahme
- 43: Blutpumpenauslass
- 44: Standfläche
- 45: Befestigungseinrichtung
- 46: Halterung
- 47: Halterungsaufnahme
- 48: Zwischensteg
- 49: Platte

## Patentansprüche

1. Koffer (12) mit einer Halteeinrichtung (1) zum Halten eines Oxygenators (2) und einer Blutpumpe (3), die einen mit einem Koffergehäuse drehfest verbindbaren Rahmen (4) zum Festlegen des Oxygenators (2) und der Blutpumpe (3) relativ zueinander aufweist, wobei der Rahmen (4) einen Rahmenabschnitt (5) zum drehfesten Verbinden mit dem Oxygenator (2) und einen anderen Rahmenabschnitt (6) zum drehfesten Verbinden mit der Blutpumpe (3) aufweist, wobei die Halteeinrichtung (1) teilweise in dem Koffergehäuse angeordnet ist und **gekennzeichnet dadurch, dass** die Halteeinrichtung (1) einen Handgriff (7) aufweist, dass der Handgriff (7) mit dem Rahmen (4) drehfest verbunden ist, und dass der Handgriff (7) außerhalb des Koffergehäuses angeordnet ist.

2. Koffer (12) mit einer Halteeinrichtung (1) nach Anspruch 1, **dadurch gekennzeichnet, dass** die Halteeinrichtung (1) mit dem Koffergehäuse (13) drehfest verbunden ist.

3. Koffer (12) nach Anspruch 1 oder 2, **dadurch gekennzeichnet, dass** das Koffergehäuse (13) einen ersten Gehäuseabschnitt (14) und einen zweiten Gehäuseabschnitt (15) aufweist, wobei der erste Gehäuseabschnitt (14) und der zweite Gehäuseabschnitt (15) wieder lösbar miteinander drehfest verbunden sind.

4. Koffer (12) nach Anspruch 3, **dadurch gekennzeichnet, dass**
a. dass der Oxygenator (2) und die Blutpumpe (3) mit dem Rahmen (4) drehfest verbunden sind oder dass
b. eine erste Aufnahme (16) des ersten Gehäuseabschnitts (14) und eine zweite Aufnahme (17) des zweiten Gehäuseabschnitts (15) einen Hohlraum bilden, in dem ein Teil des Rahmens (4) und der Oxygenator (2) und die Blutpumpe (3) angeordnet ist und dass
c. wenigstens ein Teil des Rahmens (4) und der Oxygenator (2) und die Blutpumpe (3) in dem ersten Gehäuseabschnitt (14) und dem zweiten Gehäuseabschnitt (15) wenigstens teilweise eingebettet ist.

5. Koffer (12) nach Anspruch 4, **dadurch gekennzeichnet, dass**
a. zum teilweisen Freigeben des Oxygenators ein den Oxygenator (2) aufnehmender Teil der zweiten Aufnahme (18) als Durchbruch ausgeführt ist und dass
b. zum teilweisen Freigeben der Blutpumpe ein die Blutpumpe aufnehmender anderer Teil der zweiten Aufnahme (19) als Durchbruch ausgeführt ist und dass
c. zum teilweisen Freigeben des Teils des Rahmens (4) ein den Rahmen (4) aufnehmender weiterer Teil der zweiten Aufnahme (40) als Durchbruch ausgeführt ist und dass
d. der zweite Gehäuseabschnitt (15) eine Ausnehmung (31) aufweist.

6. Koffer (12) nach Anspruch 5, **dadurch gekennzeichnet, dass** der Koffer (12) einen Verschlussdeckel (20) zum Verschließen des Teils der zweiten Aufnahme (18) und des anderen Teils der zweiten Aufnahme (19) und des weiteren Teils der zweiten Aufnahme (40) und der Ausnehmung (31) aufweist.

7. Koffer (12) nach einem der Ansprüche 3 bis 6, **dadurch gekennzeichnet, dass** der erste Gehäuseabschnitt (14) und der zweite Gehäuseabschnitt (15) eine Durchgangsöffnung (25) aufweist,
a. durch die die mit dem Oxygenator (2) und der Blutpumpe (3) fluidisch verbundene Fluidleitung aus dem Koffergehäuse (13) austritt oder durch die mehrere mit dem Oxygenator (2) und der Blutpumpe (3) fluidisch verbundene Fluidleitungen aus dem Koffergehäuse (13) austreten und
b. durch die sich ein Teil des Rahmens (4) erstreckt.

8. Koffer (12) nach einem der Ansprüche 3 bis 7, **dadurch gekennzeichnet, dass**
a. der Koffer (12) einen Fluidauslassdurchgang (21) aufweist, durch den ein Fluid aus dem ersten Gehäuseabschnitt (14) und dem zweiten Gehäuseabschnitt (15) ausströmbar ist und dass
b. der Koffer (12) eine weitere Durchgangsöffnung (22) aufweist, durch den sich wenigstens eine Abzweigleitung zum Zuführen von Blut zu einer Analyseeinrichtung erstreckt.

9. Koffer (12) nach einem der Ansprüche 1 bis 8, **dadurch gekennzeichnet, dass** die Blutpumpe (3) stromlos antreibbar ist.

10. Koffer (12) nach einem der Ansprüche 2 bis 9, **dadurch gekennzeichnet, dass** der Handgriff (7) und der Rahmen (4) einstückig ausgeführt sind.

11. Koffer (12) nach einem der Ansprüche 1 bis 10, **dadurch gekennzeichnet, dass** der Koffer (12) eine Befestigungseinrichtung (45) zum Befestigen des Koffers (12) mit einem Gegenstand aufweist.

12. Koffer (12) nach einem der Ansprüche 1 bis 11, **gekennzeichnet durch** eine Standfläche (44), die derart ausgebildet und angeordnet ist, dass ein Oxygenatoreinlass (42) in vertikaler Richtung oberhalb eines Blutpumpenauslasses (43) angeordnet ist, wenn der Koffer (12) mittels der Standfläche (44) auf eine Abstellfläche abgesetzt ist.

13. Koffer (12) nach einem der Ansprüche 1 bis 12, **dadurch gekennzeichnet, dass** wenigstens eine Fluidleitung und wenigstens eine Abzweigleitung mit einem Rückholelement zum Rückholen des aus dem Koffergehäuse erstreckten Teils der Fluidleitung und der Abzweigleitung in das Koffergehäuse verbunden sind.

14. Koffer (12) nach einem der Ansprüche 1 bis 13, **dadurch gekennzeichnet, dass** der Koffer (12) ein Set von gegeneinander auswechselbaren Oxygenatoren und ein Set von gegeneinander auswechselbaren Blutpumpen und ein Set von gegeneinander auswechselbaren Halteeinrichtungen aufweist.

15. Herz-Lungen-Maschine mit einem Koffer (12) nach einem der Ansprüche 1 bis 14, einer Gasquelle (23) und einer mit der Gasquelle (23) und dem Koffer (12) verbundenen Vorrichtung zum Steuern oder Regeln (24) eines durch die Blutpumpe (3) geförderten Blutvolumenstroms und eines durch den Oxygenator (2) strömenden Gasvolumenstroms.

## Claims

1. Case (12) with a holding device (1) for holding an oxygenator (2) and a blood pump (3), which has a frame (4) which can be connected in a rotationally fixed manner to a case housing for fixing the oxygenator (2) and the blood pump (3) relative to one another, wherein the frame (4) has a frame section (5) for connecting in a rotationally fixed manner to the oxygenator (2) and another frame section (6) for connecting in a rotationally fixed manner to the blood pump (3), wherein the holding device (1) is partially arranged in the case housing, and **characterized in that** the holding device (1) has a handle (7), that the handle (7) is connected in a rotationally fixed manner to the frame (4), and that the handle (7) is arranged outside the case housing.

2. Case (12) with a holding device (1) according to Claim 1, **characterized in that** the holding device (1) is connected to the case housing (13) in a rotationally fixed manner.

3. Case (12) according to Claim 1 or 2, **characterized in that** the case housing (13) has a first housing section (14) and a second housing section (15),
wherein the first housing section (14) and the second housing section (15) are releasably connected to one another in a rotationally fixed manner.

4. Case (12) according to Claim 3, **characterized in that**
a. the oxygenator (2) and the blood pump (3) are connected to the frame (4) in a rotationally fixed manner or that
b. a first receptacle (16) of the first housing section (14) and a second receptacle (17) of the second housing section (15) form a cavity in which a part of the frame (4) and the oxygenator (2) and the blood pump (3) are arranged and that
c. at least a part of the frame (4) and the oxygenator (2) and the blood pump (3) are at least partially embedded in the first housing section (14) and the second housing section (15).

5. Case (12) according to Claim 4, **characterized in that**
a. for partially releasing the oxygenator, a part of the second receptacle (18) accommodating the oxygenator (2) is designed as an opening and that
b. for partially releasing the blood pump, another part of the second receptacle (19) accommodating the blood pump is designed as an opening and that
c. for partially releasing the part of the frame (4), a further part of the second receptacle (40) accommodating the frame (4) is designed as an opening and that
d. the second housing section (15) has a recess (31).

6. Case (12) according to Claim 5, **characterized in that** the case (12) has a closure lid (20) for closing the part of the second receptacle (18) and the other part of the second receptacle (19) and the further part of the second receptacle (40) and the recess (31).

7. Case (12) according to one of Claims 3 to 6, **characterized in that** the first housing section (14) and the second housing section (15) have a through-opening (25),
a. through which the fluid line fluidically connected to the oxygenator (2) and the blood pump (3) exits the case housing (13) or through which a plurality of fluid lines fluidically connected to the oxygenator (2) and the blood pump (3) exit the case housing (13) and
b. through which a part of the frame (4) extends.

8. Case (12) according to one of Claims 3 to 7, **characterized in that**
a. the case (12) has a fluid outlet passage (21) through which a fluid can flow out of the first housing section (14) and the second housing section (15) and that
b. the case (12) has a further through-opening (22) through which at least one branch line for supplying blood to an analysis device extends.

9. Case (12) according to one of Claims 1 to 8, **characterized in that** the blood pump (3) can be driven without current.

10. Case (12) according to one of Claims 2 to 9, **characterized in that** the handle (7) and the frame (4) are made in one piece.

11. Case (12) according to one of Claims 1 to 10, **characterized in that** the case (12) has a fastening device (45) for fastening the case (12) to an object.

12. Case (12) according to one of Claims 1 to 11, **characterized by** a standing surface (44) which is designed and arranged such that an oxygenator inlet (42) is arranged in the vertical direction above a blood pump outlet (43) when the case (12) is placed on a storage surface by means of the standing surface (44).

13. Case (12) according to one of Claims 1 to 12, **characterized in that**
a. at least one fluid line and at least one branch line are connected to a return element for returning the part of the fluid line and the branch line extending out of the case housing into the case housing.

14. Case (12) according to one of Claims 1 to 13, **characterized in that** the case (12) has a set of interchangeable oxygenators and a set of interchangeable blood pumps and a set of interchangeable holding devices.

15. Heart-lung machine with a case (12) according to one of Claims 1 to 14, a gas source (23) and a device connected to the gas source (23) and the case (12) for controlling or regulating (24) a blood volume flow conveyed by the blood pump (3) and a gas volume flow flowing through the oxygenator (2).

## Revendications

1. Mallette (12) avec un dispositif de retenue (1) destiné à retenir un oxygénateur (2) et une pompe d'assistance circulatoire (3), qui présente un cadre (4) pouvant être relié de manière solidaire en rotation à une mallette pour fixer l'oxygénateur (2) et la pompe d'assistance circulatoire (3) l'un par rapport à l'autre, le cadre (4) présentant une partie (5) destinée à être reliée de manière solidaire en rotation à l'oxygénateur (2) et une autre partie (6) destinée à être reliée de manière solidaire en rotation à la pompe d'assistance circulatoire (3), le dispositif de retenue (1) étant partiellement disposé dans le boîtier de la mallette, et **caractérisé en ce que** le dispositif de retenue (1) présente une poignée (7), **en ce que** la poignée (7) est reliée de manière solidaire en rotation au cadre (4), et **en ce que** la poignée (7) est disposée à l'extérieur du boîtier de la mallette.

2. Mallette (12) avec un dispositif de retenue (1) selon la revendication 1, **caractérisée en ce que** le dispositif de retenue (1) est relié de manière solidaire en rotation au boîtier de la mallette (13).

3. Mallette (12) selon la revendication 1 ou 2, **caractérisée en ce que** le boîtier de la mallette (13) présente une première partie (14) et une deuxième partie (15), la première partie (14) et la deuxième partie (15) étant à nouveau reliées détachables l'une à l'autre de manière solidaire en rotation.

4. Mallette (12) selon la revendication 3, **caractérisée en ce que**
a. l'oxygénateur (2) et la pompe d'assistance circulatoire (3) sont reliés de manière solidaire en rotation au cadre (4) ou **en ce que**
b. un premier logement (16) de la première partie (14) et un deuxième logement (17) de la deuxième partie (15) forment un espace creux dans lequel une partie du cadre (4) et l'oxygénateur (2) et la pompe d'assistance circulatoire (3) sont disposés et **en ce que**
c. au moins une partie du cadre (4) et l'oxygénateur (2) et la pompe d'assistance respiratoire (3) sont au moins partiellement encastrés dans la première partie (14) et la deuxième partie (15).

5. Mallette (12) selon la revendication 4, **caractérisée en ce que**
a. pour libérer partiellement l'oxygénateur, une partie du deuxième logement (18) recevant l'oxygénateur (2) est réalisée sous forme de passage et **en ce que**
b. pour libérer partiellement la pompe d'assistance circulatoire, une autre partie du deuxième logement (19) recevant la pompe d'assistance circulatoire est réalisée sous forme de passage et **en ce que**
c. pour libérer partiellement la partie du cadre (4), une autre partie du deuxième logement (40) recevant le cadre (4) est réalisée sous forme de passage et **en ce que**
d. la deuxième partie (15) présente un évidement (31).

6. Mallette (12) selon la revendication 5, **caractérisée en ce que** la mallette (12) présente un couvercle de fermeture (20) pour fermer la partie du deuxième logement (18) et l'autre partie du deuxième logement (19) et l'autre partie du deuxième logement (40) et l'évidement (31).

7. Mallette (12) selon l'une des revendications 3 à 6, **caractérisée en ce que** la première partie (14) et la deuxième partie (15) présentent une ouverture de passage (25),
a. par laquelle la conduite de fluide reliée de manière fluidique à l'oxygénateur (2) et à la pompe d'assistance circulatoire (3) sort du boîtier de la mallette (13) ou par laquelle plusieurs conduites de fluide reliées de manière fluidique à l'oxygénateur (2) et à la pompe d'assistance circulatoire (3) sortent du boîtier de la mallette (13) et
b. à travers laquelle s'étend une partie du cadre (4).

8. Mallette (12) selon l'une des revendications 3 à 7, **caractérisée en ce que**
a. la mallette (12) comporte un passage de sortie de fluide (21) à travers lequel un fluide peut être évacué de la première partie (14) et de la deuxième partie (15), et **en ce que**
b. la mallette (12) présente une autre ouverture de passage (22) à travers laquelle s'étend au moins une conduite de dérivation pour l'amenée de sang à un dispositif d'analyse.

9. Mallette (12) selon l'une des revendications 1 à 8, **caractérisée en ce que** la pompe d'assistance circulatoire (3) peut être entraînée sans courant.

10. Mallette (12) selon l'une des revendications 2 à 9, **caractérisée en ce que** la poignée (7) et le cadre (4) sont réalisés d'une seule pièce.

11. Mallette (12) selon l'une des revendications 1 à 10, **caractérisée en ce que** la mallette (12) présente un dispositif de fixation (45) pour fixer la mallette (12) à un objet.

12. Mallette (12) selon l'une des revendications 1 à 11, **caractérisée par** une surface d'appui (44) qui est formée et disposée de telle sorte qu'une entrée d'oxygénateur (42) est disposée dans la direction verticale au-dessus d'une sortie de pompe d'assistance circulatoire (43) lorsque la mallette (12) est déposée sur un reposoir au moyen de la surface d'appui (44).

13. Mallette (12) selon l'une des revendications 1 à 12, **caractérisée en ce que**
a. au moins une conduite de fluide et au moins une conduite de dérivation sont reliés à un élément de récupération pour récupérer la partie de la conduite de fluide et de la conduite de dérivation s'étendant hors du boîtier de la mallette dans le boîtier de la mallette.

14. Mallette (12) selon l'une des revendications 1 à 13, **caractérisée en ce que** la mallette (12) présente un ensemble d'oxygénateurs interchangeables et un ensemble de pompes d'assistance circulatoire interchangeables et un ensemble de dispositifs de retenue interchangeables.

15. Machine cardio-pulmonaire avec une mallette (12) selon l'une des revendications 1 à 14, une source de gaz (23) et un dispositif de commande ou de régulation (24), relié à la source de gaz (23) et à la mallette (12), d'un débit volumique sanguin refoulé par la pompe d'assistance circulatoire (3) et d'un débit volumique gazeux circulant dans l'oxygénateur (2).
